(19) Europäisches Patentamt
European Patent Office
Office européen des brevets

(11) EP 4 382 174 A1

(12) EUROPEAN PATENT APPLICATION
published in accordance with Art. 153(4) EPC

(43) Date of publication:
**12.06.2024 Bulletin 2024/24**

(21) Application number: **22852902.0**

(22) Date of filing: **26.07.2022**

(51) International Patent Classification (IPC):
***A61Q 5/06*** *(2006.01)* ***A61Q 5/12*** *(2006.01)*
***A61K 8/31*** *(2006.01)* ***A61K 8/34*** *(2006.01)*
***A61K 8/81*** *(2006.01)* ***A61K 8/891*** *(2006.01)*

(52) Cooperative Patent Classification (CPC):
**A61K 8/31; A61K 8/34; A61K 8/58; A61K 8/73; A61K 8/81; A61K 8/89; A61K 8/891; A61K 8/91; A61Q 5/00; A61Q 5/06; A61Q 5/12**

(86) International application number:
**PCT/JP2022/028785**

(87) International publication number:
**WO 2023/013479 (09.02.2023 Gazette 2023/06)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.08.2021 JP 2021126796**

(71) Applicant: **Kao Corporation**
**Tokyo 103-8210 (JP)**

(72) Inventor: **MAEKAWA, Tomoka**
**Tokyo 131-8501 (JP)**

(74) Representative: **Hoffmann Eitle**
**Patent- und Rechtsanwälte PartmbB**
**Arabellastraße 30**
**81925 München (DE)**

# (54) COSMETIC COMPOSITION

(57) A cosmetic composition containing a component (A): a film-forming polymer containing an M unit represented by $(R^1)_3SiO_{1/2}$ and a Q unit represented by $SiO_{4/2}$ (in the formula, $R^1$ represents a hydrocarbon group having 1 or more and 12 or less carbon atoms and optionally substituted with fluorine, or a hydroxy group, and plural $R^1$'s can be the same as or different from each other), a component (B): a film-forming polymer other than the component (A), and a component (C): a volatile solvent, wherein the component (C) has a content of 50% by mass or more.

EP 4 382 174 A1

## Description

Field of the Invention

The present invention relates to a cosmetic composition.

Background of the Invention

It is known that hair is damaged and difficult to handle by external factors in daily life, and chemical treatment such as perm and color. In order to solve such a situation, a conditioning agent or a styling agent is used to impart gloss or smoothness to hair.

However, many of these effects are lost by one shampooing and products capable of sustaining the effects are desired. Therefore, in recent years, in the field of cosmetics, a technique for forming a hydrophobic film has been studied as a method of imparting gloss, smooth feel, etc. to the skin or hair.

For example, JP 2015-515981 A (Patent Literature 1) discloses that a composition containing a continuous aqueous phase, and a discontinuous phase that contains a plasticized MQ-type silicone resin and/or a derivative thereof, and/or a plasticized MT-type silicone resin and/or a derivative thereof, in which the discontinuous phase is emulsified in the aqueous phase, is useful as a haircare product for treating split ends.

As one hair dyeing technique, there is known a temporary hair dye technique of using a pigment as a colorant and forming a film that contains the colorant on hair for dyeing the hair. For example, JP H10-265354 A (Patent Literature 2) discloses that a hair dye containing a volatile oil, a water-repellent polymer dissolving in the volatile oil, a powder, and a nonvolatile oil compatible with the volatile oil, in which at least a part of the powder is a color pigment, is applicable in a simple manner and secures good color duration not causing secondary adhesion after application.

Summary of the Invention

The present invention relates to the following [1] to [7].

[1] A cosmetic composition containing

a component (A): a film-forming polymer containing an M unit represented by $(R^1)_3SiO_{1/2}$ and a Q unit represented by $SiO_{4/2}$ (wherein $R^1$ represents a hydrocarbon group having 1 or more and 12 or less carbon atoms and optionally substituted with fluorine, or a hydroxy group, and plural $R^1$'s can be the same as or different from each other),
a component (B): a film-forming polymer other than the component (A), and
a component (C): a volatile solvent,

wherein the component (C) has a content of 50% by mass or more.
[2] A hair cosmetic composition composed of the cosmetic composition according to the above [1].
[3] A hair dye composition composed of the cosmetic composition according to the above [1].
[4] A method for treating a keratin substance, including a step of applying the cosmetic composition according to the above [1] to a keratin substance and then drying it.
[5] A method for treating hair, including a step of applying the hair cosmetic composition according to the above [2] to hair and then drying it.
[6] A method for dyeing hair, comprising a step of applying the hair dye composition according to the above [3] to hair and then drying it.
[7] A cosmetic kit provided with two or more compositions,
wherein the following components (A) to (C) are contained in a cosmetic composition obtained by mixing the compositions:

a component (A): a film-forming polymer containing an M unit represented by $(R^1)_3SiO_{1/2}$ and a Q unit represented by $SiO_{4/2}$ (wherein $R^1$ represents a hydrocarbon group having 1 or more and 12 or less carbon atoms and optionally substituted with fluorine, or a hydroxy group, and plural $R^1$'s can be the same as or different from each other),
a component (B): a film-forming polymer other than the component (A), and
a component (C): a volatile solvent.

Detailed Description of the Invention

There is a desire for a cosmetic composition that can impart a smooth feel to keratin substances such as skin and hair, and that has high washing durability. Regarding washing durability, for example, the temporary hair dye described in Patent Literature 2 is required to have difficulty of discoloration even if hair is washed after dyeing.
In addition, it is important for cosmetic compositions applied to the skin or hair to be easy to spread over a wide area from the viewpoint of performance and handleability. However, with conventional cosmetic compositions, it has been difficult to simultaneously provide a smooth feel and washing durability and ease of application.
An object of the present invention is to provide a cosmetic composition that can impart a smooth feel when applied to keratin substances such as skin and hair, has excellent washing durability, and is easy to spread over a wide area.
The present inventors have discovered that a cosmetic composition containing at least two types of predetermined film-forming polymers and a predetermined amount or more of a volatile solvent can solve the above problems, and have completed the present invention.
According to the present invention, it is possible to provide a cosmetic composition that can impart a smooth feel when applied to keratin substances such as skin and hair, has excellent washing durability, and is easy to spread over a wide area. Further, when the cosmetic composition is used as a hair dye composition, it can impart a smooth feel and ease of applying and spreading to the hair with good color duration and little discoloration by shampooing.

[Definition]

"Polymer" used in the present specification means a compound corresponding to a repetition of one or plural units (these units are derived from a compound known as a monomer). This or these units are repeated at least two times, preferably at least three times.
"Hair" used in the present specification means mainly head hair.
"Hydrophobic" used in the present specification means that a solubility in water of a substance is less than 1% by mass at 25°C.
"Film formation" used in the present specification means that, when applied to a substrate, a film is left thereon.
"Volatile" used in the present specification means a substance having a boiling point of 260°C or lower under normal pressure.

[Cosmetic Composition]

The cosmetic composition of the present invention contains

a component (A): a film-forming polymer containing an M unit represented by $(R^1)_3SiO_{1/2}$ and a Q unit represented by $SiO_{4/2}$ (wherein $R^1$ represents a hydrocarbon group having 1 or more and 12 or less carbon atoms and optionally substituted with fluorine, or a hydroxy group, and plural $R^1$'s can be the same as or different from each other),
a component (B): a film-forming polymer other than the component (A), and
a component (C): a volatile solvent,

and the component (C) has a content of 50% by mass or more. Hereinafter, the "M unit represented by $(R^1)_3SiO_{1/2}$ and the Q unit represented by $SiO_{4/2}$" will also be referred to as "MQ unit".
With the aforementioned structure, the cosmetic composition of the present invention can impart a smooth feel when applied to keratin substances such as skin and hair, has excellent washing durability, and is easy to apply and spread over a wide area. Furthermore, when the cosmetic composition is used as a hair dye composition, it is possible to impart a smooth feel and ease of applying and spreading to the hair with good color duration and little discoloration by shampooing.
Hereinafter, in the present specification, the fact that the treatment effect persists even after washing the keratin substance treated with the cosmetic composition, and in the case of a hair dye composition, there is little discoloration due to shampooing is also referred to as "washing durability" as appropriate.
The reason why the cosmetic composition of the present invention exhibits the above-mentioned effects is, though not clear, presumed as follows.
The cosmetic composition of the present invention containing the component (A) and the component (B) that are hydrophobic film-forming agents is, when applied to the surface of a keratin substance, able to form a hydrophobic film.
Since the component (A) contains rigid MQ units, it can form a hard film. On the other hand, since the component (A) has low toughness, the formed film tends to be brittle and also difficult to impart a smooth feel.
It is considered that, since the component (B) usually has higher toughness than the component (A), in the

cosmetic composition of the present invention, the use of the component (B) increases the flexibility of the film to be formed, and as a result, washing durability will be improved. Furthermore, by containing a predetermined amount or more of the component (C) in the cosmetic composition, the cosmetic composition becomes easier to apply and spread, making it possible to form a uniform film. Even when the content of the components (A) and (B), which are film-forming polymers, is low, it could be possible to impart a smooth feel to the surface of the keratin substance to which the cosmetic composition has been applied, and to improve washing durability.

In the case where a functional powder to be mentioned hereinunder is blended in the cosmetic composition, the functional powder can be kept in the film to enhance various functions and sustainability thereof.

The mechanism of the action of the present invention is not limited to the above.

Components contained in the cosmetic composition of the present invention are described below.

<Component (A): Film-forming Polymer>

The cosmetic composition of the present invention contains a film-forming polymer containing, as the component (A), an M unit represented by $(R^1)_3SiO_{1/2}$ and a Q unit represented by $SiO_{4/2}$ (wherein $R^1$ represents a hydrocarbon group having 1 or more and 12 or less carbon atoms and optionally substituted with fluorine, or a hydroxy group, and plural $R^1$'s can be the same as or different from each other).

Containing the component (A), the cosmetic composition of the present invention is, when applied to a keratin substance such as skin and hair, able to form a hard hydrophobic film. In the case where a functional powder to be mentioned hereinunder is blended in the cosmetic composition, the functional powder can be held in the film to improve various functions and sustainability thereof.

In the M unit, $R^1$ represents a hydrocarbon group having 1 or more and 12 or less carbon atoms and optionally substituted with fluorine, or a hydroxy group. The carbon number of the hydrocarbon group is, from the viewpoint of improving film formability and durability, 1 or more and preferably 9 or less, more preferably 6 or less, and even preferably 4 or less.

The hydrocarbon group may be any of an aliphatic group or an aromatic group, and examples thereof include an alkyl group, an alkenyl group, an aryl group and an aralkyl group. The alkyl group and the alkenyl group may be linear or branched.

Among the above, from the viewpoint of availability and stability, the hydrocarbon group is preferably an alkyl group, an aryl group or an aralkyl group.

The alkyl group includes a methyl group, an ethyl group, an n-propyl group, an isopropyl group, various butyl groups, various pentyl groups, various hexyl groups, various heptyl groups, various octyl groups, various nonyl group, various decyl groups, various undecyl groups, and various dodecyl groups. The word "various" means a linear or branched hydrocarbon group, and for example, "various butyl groups" include "an n-butyl group, a secbutyl group, an isobutyl group and a tert-butyl group".

The aryl group includes a phenyl group, a toluyl group, a dimethylphenyl group, and a naphthyl group, and is preferably a phenyl group.

The aralkyl group includes a benzyl group, a phenylethyl group, a phenylpropyl group, and a phenylbutyl group, and is preferably a phenylpropyl group.

In the case where $R^1$ is substituted with fluorine, at least one hydrogen atom of the hydrocarbon group may be substituted with a fluorine atom.

$R^1$ is, from the viewpoint of improving film formability and washing durability, preferably an alkyl group having 1 or more and 12 or less carbon atoms and optionally substituted with fluorine, an aryl group having 6 or more and 12 or less carbon atoms, or an aralkyl group having 7 or more and 12 or less carbon atoms, more preferably an alkyl group having 1 or more and 8 or less carbon atoms and optionally substituted with fluorine, or a phenyl group, and even preferably an alkyl group having 1 or more and 6 or less carbon atoms and optionally substituted with fluorine, or a phenyl group. The fluorine-substituted alkyl group is preferably a group represented by $CF_3$-R-, wherein R represents an alkylene group having 2 or more and 7 or less carbon atoms, and preferably 2 or more and 5 or less carbon atoms.

$R^1$ is more preferably a trifluoropropyl group, an alkyl group having 1 or more and 4 or less carbon atoms, or a phenyl group, even preferably a trifluoropropyl group, a methyl group, an ethyl group, an n-propyl group, an isopropyl group, or an n-butyl group, even more preferably a trifluoropropyl group, a methyl group, or an n-propyl group, and still preferably a methyl group.

The component (A) may be any film-forming polymer having the above-mentioned MQ units, and is preferably a hydrophobic film-forming polymer having MQ units.

The component (A) may further contain a D unit represented by $(R^1)_2SiO_{2/2}$ ($R^1$ is the same as above) from the viewpoint of improving film formability and washing durability.

However, from the viewpoint of obtaining a synergistic effect by using the component (A) and the component (B), the component (A) preferably does not substantially contain a T unit represented by $R^1SiO_{3/2}$ ($R^1$ is the same as

above). "Substantially not containing XX" means that the constituent ratio of XX in the silicone resin is less than 1 mol%.

From the viewpoint of improving film formability and washing durability, the component (A) is preferably a silicone resin represented by an average formula $(R^1)_mSiO_{(4-m)/2}$ (wherein $R^1$ represents a hydrocarbon group having 1 or more and 12 or less carbon atoms and optionally substituted with fluorine, or a hydroxy group, plural $R^1$'s can be the same as or different from each other, and m is an average number, representing a number of more than 0 and less than 3) and containing an M unit represented by $(R^1)_3SiO_{1/2}$ and a Q unit represented by $SiO_{4/2}$.

The silicone resin represented by the above average formula and containing the Q unit has a crosslinked structure in the molecule. It is thought that by having the structure, it is possible to form a film having higher washing durability. The silicone resin does not contain cured polyorganosiloxane powder, which is infusible and has no softening point, and is generally insoluble in organic solvents.

The component (A) is, from the viewpoint of improving film formability and washing durability, more preferably a silicone resin represented by $[SiO_{4/2}]_c[(R^1)_3SiO_{1/2}]_d$ wherein c and d each are an average repeating unit number and c>0 and d>0.

The component (A) includes trimethylsiloxysilicate, phenylpropyldimethylsiloxysilicate, fluorine-modified alkyldimethylsiloxysilicates, and crosspolymers produced by crosslinking these siloxysilicates with dimethiconol, etc., and one or two or more of these can be used. Fluorine-modified alkyldimethylsiloxysilicates include trifluoroalkyldimethyltrimethylsiloxysilicate, such as trifluoropropyldimethyltrimethylsiloxysilicate of, as ICNI nomenclature, trifluoropropyldimethyl/trimethylsiloxysilicate. Crosspolymers produced by crosslinking siloxysilicates with dimethiconol, etc. include, as ICNI nomenclature, (trimethylsiloxysilicate/dimethiconol) crosspolymer.

Above all, from the viewpoint of improving film formability and washing durability, the component (A) is preferably one or more selected from the group consisting of trimethylsiloxysilicate, trifluoropropyldimethyltrimethylsiloxysilicate and (trimethylsiloxysilicate/dimethiconol) crosspolymer, more preferably one or more selected from the group consisting of trimethylsiloxysilicate, and trifluoropropyldimethyltrimethylsiloxysilicate, and even preferably trimethylsiloxysilicate.

Commercial products of trimethylsiloxysilicate of the component (A) include KF-7312J (50 mass% decamethylcyclopentasiloxane solution), KF-9021 (50 mass% decamethylcyclopentasiloxane solution), X-21-5249(50 mass% decamethylcyclopentasiloxane solution), X-21-5595 (60 mass% isododecane solution), and X-21-5616 (60 mass% isododecane solution) (all by Shin-Etsu Chemical Industry Co., Ltd.), SS4267 (35 mass% dimethylpolysiloxane solution), SR1000, SS4230 (45 mass% cyclopentasiloxane solution), SS4267 (35 mass% dimethylpolysiloxane solution), and Silsoft 74 (75 mass% isododecane solution) (all by Momentive Performance Materials Corporation), BY11-018 (30 mass% cyclopentasiloxane solution), and MQ-1600 Solid Resin (both by Dow Toray Corporation), and BELSIL TMS 803 (by Wacker Asahi Kasei Silicone Co., Ltd.).

Commercial products of phenylpropyldimethylsiloxysilicate include SilShine 151 (by Momentive Performance Materials Corporation).

Commercial products of fluorine-modified alkyldimethylsiloxysilicates include, XS66-B8226 (50 mass% cyclopentasiloxane solution), XS66-C1191, and XS66-B8636 (50 mass% dimethicone solution) (all by Momentive Performance Materials Corporation), as INCI nomenclature, trifluoropropyldimethyl/trimethylsiloxysilicate.

Commercial products of trimethylsiloxysilicate crosspolymer include DOWSIL FC-5002 IDD Resin Gum (40 mass% isododecane solution of (trimethylsiloxysilicate/dimethiconol) crosspolymer) (by Dow Toray Corporation).

<Component (B): Film-forming Polymer other than Component (A)>

The cosmetic composition of the present invention contains, as the component (B), a film-forming polymer other than the component (A). It is thought that by using the component (B), the flexibility of the film containing the component (A) can be increased and washing durability can be improved.

From the viewpoint of compatibility with the component (A) and from the viewpoint of improving washing durability of the film to be formed, the component (B) is preferably a hydrophobic film-forming polymer.

As the hydrophobic film-forming polymer, any of silicone-based polymers and non-silicone-based polymers can be used, and these may be used in combination. The silicone-based polymer of the component (B) refers to a film-forming polymer that has a silicone structure other than the component (A), and the non-silicone-based polymer refers to a film-forming polymer that does not have a silicone structure.

The film-forming polymer used as the component (B) includes one or more selected from the group consisting of the following components (B1) to (B6). Of the following, silicone-based polymers are components (B1) to (B5), and non-silicone-based polymer is component (B6).

(B1) A silicone resin containing a T unit represented by $R^1SiO_{3/2}$ and substantially not containing a Q unit represented by $SiO_{4/2}$ ($R^1$ is the same as above).

(B2) An acryl silicone polymer.

(B3) A silicone-modified alicyclic structure-containing polymer.

(B4) A silicone-modified pullulan.
(B5) A polyurea/urethane silicone.
(B6) A non-silicone-based polymer with a glass transition temperature of 200°C or lower.

(Component (B 1): Silicone Resin)

The component (B 1) is a silicone resin containing a T unit represented by $R^1SiO_{3/2}$ and substantially not containing a Q unit represented by $SiO_{4/2}$ ($R^1$ is the same as above).
The component (B 1) preferably further contains one or more units selected from the group consisting of an M unit represented by $(R^1)_3SiO_{1/2}$ and a D unit represented by $(R^1)_2SiO_{2/2}$. $R^1$ is the same as above.
From the viewpoint of increasing the flexibility of the film to be formed and improving washing durability, the component (B 1) is preferably a silicone resin represented by $[R^1SiO_{3/2}]_a[(R^1)_3SiO_{1/2}]_b$ (a and b each are an average repeating unit number and a>0 and b≥0) containing a T unit and optionally containing an M unit. "Substantially not containing XX" means that the constituent ratio of XX in the silicone resin is less than 1 mol%.
$R^1$ is the same as above, and is preferably an alkyl group having 1 or more and 4 or less carbon atoms or a phenyl group, more preferably a methyl group, an ethyl group, an n-propyl group or an isopropyl group, and even preferably a methyl group, an n-propyl group or an isopropyl group.
The component (B 1) includes polysilsesquioxanes such as polymethylsilsesquioxane, polypropylsilsesquioxane, polyphenylsilsesquioxane, polymethylphenylsilsesquioxane, and fluorine-modified alkyldimethylpolysilsesquioxane, and among these, one or two or more can be used. Fluorine-modified alkyldimethylpolysilsesquioxanes include, as INCI nomenclature, (trifluoropropyldimethylsiloxy/trimethylsiloxy) silsesquioxane.
Above all, from the viewpoint of increasing the flexibility of the film to be formed and improving washing durability, the component (B 1) is preferably one or more selected from the group consisting of polymethylsilsesquioxane and polypropylsilsesquioxane, and more preferably polypropylsilsesquioxane.
Commercial products of the component (B 1) include SilForm Flexible Resin (polymethylsilsesquioxane), and SilForm FR-5 (polydimethylsiloxane solution of (trifluoropropyldimethylsiloxy/trimethylsiloxy)silsesquioxane) (both by Momentive Performance Materials Corporation), DOWSIL 680 ID Fluid (isododecane solution of 75 mass% polypropylsilsesquioxane) (by Dow Toray Corporation), SR-21 (polyphenylsilsesquioxane), SR-23 (polyphenylsilsesquioxane), and SR-33 (polymethylphenylsilsesquioxane) (all by Konishi Chemical Industry Co., Ltd.).

(Component (B2): Acryl Silicone Polymer)

The component (B2) includes an acrylic polymer having a carbosiloxane dendrimer structure in the side chain, an acryl-silicone graft copolymer, and a graft-type copolymer or alternate block-type copolymer where a structural unit of a polysiloxane group and a structural unit of a polymer of an unsaturated monomer bond via a sulfide bond.
The acrylic polymer having a carbosiloxane dendrimer structure in the side chain includes a silicone dendrimer-acryl copolymer, and for example, can be produced according to the production method described in JP H11-1530 A and JP 2000-63225 A.
The acrylic polymer having a carbosiloxane dendrimer structure in the side chain is preferably, as INCI nomenclature, (acrylates/polytrimethylsiloxymethacrylate) copolymer. Commercial products thereof include DOWSIL FA 4001 CM Silicone Acrylate (30 mass% decamethylcyclopentasiloxane solution), DOWSIL FA 4002 ID Silicone Acrylate (40 mass% isododecane solution), DOWSIL FA 4003 DM Silicone Acrylate (40 mass% dimethicone solution), and DOWSIL FA 4004 ID Silicone Acrylate (40 mass% isododecane solution) (all by Dow Toray Corporation).
The acryl-silicone graft copolymer includes a radical polymer of an organopolysiloxane compound having a radical polymerizable group at one terminal of the molecular chain and a radical polymerizable monomer mainly composed of an acrylate and/or a methacrylate.
The radical polymer of an organopolysiloxane compound having a radical polymerizable group at one terminal of the molecular chain and a radical polymerizable monomer mainly composed of an acrylate and/or a methacrylate usable here includes those described in JP H2-25411 A and JP H2-132141 A, and acryl-silicone graft copolymers described in JP H3-162442 A and JP 2003-104825 A.
The acryl-silicone graft copolymer is preferably, as INCI nomenclature, (acrylates/dimethicone) copolymer. Commercial products thereof include KP-545 (30 mass% decamethylcyclopentasiloxane solution), KP-549 (40 mass% methyl trimethicone solution), and KP-550 (40 mass% isododecane solution) (all by Shin-Etsu Chemical Industry Co., Ltd.).
The graft-type copolymer or alternate block-type copolymer where a structural unit of a polysiloxane group and a structural unit of a polymer of an unsaturated monomer bond via a sulfide bond includes graft-type copolymers or alternate block-type copolymers described in JP H6-92825 A.
Above all, from the viewpoint of increasing the flexibility of the film to be formed and improving washing durability,

the component (B2) is preferably one or more selected from the group consisting of an acrylic polymer having a carbosiloxane dendrimer structure in the side chain and an acryl-silicone graft copolymer, and more preferably (acrylates/dimethicone) copolymer.

(Component (B3): Silicone-Modified Alicyclic Structure-Containing Polymer)

Examples of the silicone-modified alicyclic structure-containing polymer, which is the component (B3), include silicone-modified cyclic polyolefins, and preferred examples thereof include silicone-modified polynorbornenes represented by the following general formula (B3-1).

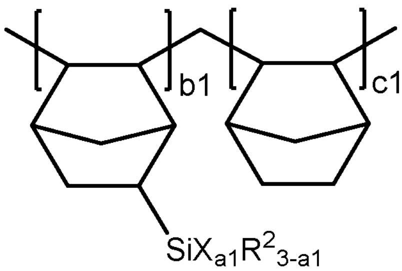

(B3-1)

wherein $R^2$ each independently represents an alkyl group having 1 or more and 12 or less carbon atoms, X represents a group represented by the following formula (i), a1 is an integer of 1 or more and 3 or less, b1 and c1 each are a repeating unit number, and are each independently an integer of 1 or more.

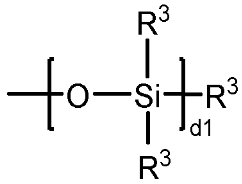

(i)

wherein $R^3$ each independently represents a hydrocarbon group having 1 or more and 12 or less carbon atoms, and d1 is an integer of 1 or more and 5 or less.

In the general formula (B3-1), $R^2$ is preferably a methyl group, an ethyl group, an n-propyl group, a butyl group or a pentyl group, and more preferably a methyl group.

X is a group represented by the formula (i), and in the formula (i), $R^3$ each are independently a hydrocarbon group having 1 or more and 12 or less carbon atoms. $R^3$ is preferably an alkyl group having 1 or more and 12 or less carbon atoms or a phenyl group, more preferably an alkyl group having 1 or more and 3 or less carbon atoms, and even preferably a methyl group. d1 is an integer of 1 or more and 5 or less, and is, from the viewpoint of versatility, preferably d1 = 1. Specifically, X is preferably a trimethylsiloxy group.

a1 is an integer of 1 or more and 3 or less, and, for example, in the polymer, a repeating unit of a1 = 2 and a repeating unit of a1 = 3 may exist as mixed. From the viewpoint of versatility, a1 is preferably 3.

The proportion of b1 and c1 in the general formula (B3-1) is preferably b1/c1 = 20/80 or more and 90/10 or less (mol/mol), more preferably 30/70 or more and 80/20 or less (mol/mol), and even preferably 50/50 or more and 70/30 or less (mol/mol). The proportion of b1 and c1 can be determined by $^1$H-NMR measurement.

The silicone-modified polynorbornene is preferably a silicone-modified polynorbornene represented by the following formula (B3-2).

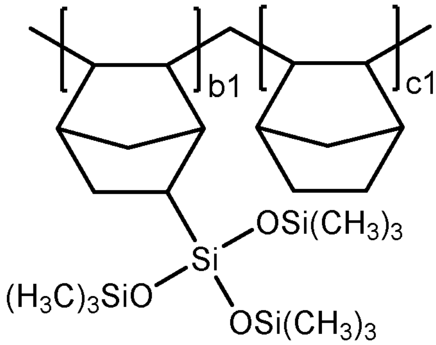

(B3-2)

wherein b1 and c1 are the same as above.

The silicone-modified polynorbornene represented by the formula (B3-2) includes a compound of, as INCI nomenclature, (norbornene/tris(trimethylsiloxy)silylnorbornene) copolymer.

Commercial products of the silicone-modified polynorbornene include NBN-30-ID (isododecane solution of (norbornene/tris(trimethylsiloxy)silylnorbornene) copolymer) (by Shin-Etsu Chemical Industry Co., Ltd.).

(Component (B4): Silicone-Modified Pullulan)

The component (B4) includes a pullulan having a silicone structure in the side chain, and specifically preferred is a silicone-modified pullulan in which at least a part of the hydrogen atoms of the OH groups in pullulan are substituted with a group represented by the following general formula (ii).

$$-R^4\text{-}SiX_{a1}R^2_{3\text{-}a1} \qquad \text{(ii)}$$

wherein $R^4$ represents a single bond or a divalent organic group, and $R^2$, X and a1 are the same as above. From the viewpoint of versatility, X is preferably a trimethylsiloxyl group, and a1 is preferably 3.

In the general formula (ii), $R^4$ is preferably a divalent organic group, more preferably a divalent group represented by the following general formula (iii) or (iv), and even preferably a divalent group represented by the following general formula (iv).

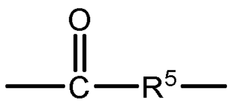

(iii)

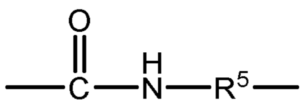

(iv)

wherein $R^5$ represents an alkylene group having 1 or more and 10 or less carbon atoms, and examples thereof include a methylene group, an ethylene group, a trimethylene group, a propylene group and a butylene group. Among these, preferred are an ethylene group, a trimethylene group and a propylene group; and more preferred are a trimethylene group or a propylene group.

Commercial products of the silicone-modified pullulan include TSPL-30-ID (isododecane solution of tri(trimethylsiloxy)silylpropylcarbamate pullulan), and TSPL-30-D5 (cyclopentasiloxane solution of tri(trimethylsiloxy)silylpropylcarbamate pullulan) (both by Shin-Etsu Chemical Industry Co., Ltd.).

(Component (B5): Polyurea/Urethane Silicone)

The component (B5) includes a polysiloxane/polyurea/polyurethane block terpolymer. For example, it is a dimethylpolysiloxane/urea copolymer of "polyurea-dimethicone" as INCI nomenclature.

The polymer can be produced by copolymerization of an $\alpha,\omega$-aminosilicone and a diisocyanate. Examples of commercial products of the polyurea/urethane silicone include "Wacker-Belsil UD 60", "Wacker-Belsil UD 80", "Wacker-Belsil UD 140" and "Wacker-Belsil UD 200" (all by Wacker Corporation).

(Component (B6): Non-silicone-based Polymer with Glass Transition Temperature of 200°C or Lower)

For the component (B6), any film-forming polymer may be used without particular limitation as long as it has a glass transition temperature (Tg) of 200°C or lower, preferably 150°C or lower, more preferably 100°C or lower, even preferably 60°C or lower, and even more preferably 5°C or lower, and does not contain a silicone structure.

The glass transition temperature of the component (B6) can be measured using a differential scanning calorimeter (DSC), and specifically can be measured by the method described in Examples. When two or more Tg peaks are observed in the DSC measurement, at least one Tg peak should be within the above range.

Examples of the component (B6) include, from the viewpoint of use in cosmetic compositions and from the viewpoint of increasing the flexibility of the film to be formed and improving washing durability, a non-silicone-based cationic polymer, a non-silicone-based anionic polymer, a non-silicone-based nonionic polymer, and a non-silicone-based amphoteric polymer, all of which have a Tg within the above range. These polymers are preferably hydrophobic polymers, and from the viewpoint of improving washing durability, are more preferably non-crosslinked polymers.

Examples of the cationic polymer include vinylpyrrolidone/N,N-dimethylaminoethyl methacrylic acid copolymer diethyl sulfate (Polyquaternium-11) (Gafquat 734 (manufactured by Ashland Specialty Ingredients), H.C. Polymer (manufactured by Osaka Organic Chemical Industry Ltd.), vinylpyrrolidone/dimethylaminopropylmethacrylamide/lauryldimethylaminopropylmethacryl amide copolymer (Styleze W-20 (manufactured by Ashland Specialty Ingredients)), polydimethylmethylenepiperidinium chloride (Merquat 100 (manufactured by Lubrizol)), dimethyldiallylammonium chloride/acrylamide copolymer (Merquat 550 (manufactured by Lubrizol)), vinylpyrrolidone/dimethylaminoethyl methacrylate copolymer (Gafquat 440 (manufactured by Ashland Specialty Ingredients)), ammonium-modified hydroxyethyl cellulose (SoftCat SL-30 polymer (manufactured by Dow Chemical)), and acrylamide/DMAPA acrylate/methoxy PEG methacrylate copolymer.

Examples of the anionic polymer include (acrylates/diacetone acrylamide) copolymer (Plas Cize L-9540B, Plas Cize L-9600U, and Plas Cize L-53, etc. (all manufactured by Goo Chemical Co., Ltd.)), (acrylates/alkyl acrylate (C1-18)/alkyl (C1-8) acrylamide) copolymer (Plas Cize L-9909B, and Plas Cize L-9900 (both manufactured by Goo Chemical Co., Ltd.)), alkyl acrylate/octylacrylamide copolymer (Dermacryl 79 (manufactured by Akzo Nobel)), vinyl acetate/crotonic acid/vinyl neodecanoate copolymer (RESYN 28-2930 (manufactured by Akzo Nobel)), acrylic acid/acrylic acid amide/ethyl acrylate copolymer (Ultrahold 8, and Ultrahold Strong (both manufactured by BASF)), alkyl acrylate copolymer (Aniset NF-1000, and Aniset HS-300, etc. (all manufactured by Osaka Organic Chemical Industry Ltd.)), and isophorone diisocyanate/dimethylolpropionic acid/(polyoxyethylene/polyoxypropylene) 4,4'-isopropylidene diphenol copolymer (DynamX (manufactured by Akzo Nobel)).

Examples of the nonionic polymer include (acrylates/methoxy methacrylate PEG-23) copolymer (Plas Cize L-188K (manufactured by Goo Chemical Co., Ltd.)), (dimethylacrylamide/hydroxyethyl acrylate/methoxyethyl acrylate) copolymer (Plas Cize L-2700, and Plas Cize L-2714 (both manufactured by Goo Chemical Co., Ltd.)), (hydroxyethyl acrylate/methoxyethyl acrylate) copolymer (Plas Cize L-2200 (manufactured by Goo Chemical Co., Ltd.)), polyvinylpyrrolidone (Luviskol K17, Luviskol K30, and Luviskol K90 (all manufactured by BASF)), vinylpyrrolidone/vinyl acetate copolymer (Luviskol VA73E, and Luviskol 37E (both manufactured by BASF)), vinyl methyl ether/alkyl maleate copolymer (Gantretz A-425, and Gantrez ES-225 (both manufactured by Ashland Specialty Ingredients)), vinylpyrrolidone/methacrylamide/vinylimidazole copolymer (Luviset Clear (manufactured by BASF)), and polyvinylcaprolactam (Luviskol Plus (manufactured by BASF)).

Examples of the amphoteric polymer include acrylate/lauryl acrylate/stearyl acrylate/ethylamine oxide methacrylate copolymer (Diaformer Z651 (manufactured by Mitsubishi Chemical Corporation)), methacryloyloxyethyl carboxybetaine/alkyl methacrylate copolymer (Yukaformer M75, and Yukaformer R205 (both manufactured by Mitsubishi Chemical Corporation)), octylacrylamide/hydroxypropyl acrylate/butylaminoethyl methacrylate copolymer (Anformer 28-4910 (manufactured by Akzo Nobel)), and acrylic acid octylamide/hydroxypropyl acrylate/butylaminoethyl methacrylate copolymer (Anformer SH30 (manufactured by Akzo Nobel)).

One or two or more can be used as the component (B).

Among the above, from the viewpoint of increasing the flexibility of the film to be formed and improving washing durability, the component (B) is preferably one or more selected from the group consisting of the components (B1) to (B6), more preferably one or more selected from the group consisting of the component (B 1), the component (B2) and the component (B6), even preferably one or more selected from the group consisting of the component (B 1) and the component (B2), and even more preferably the component (B2).

More specifically, from the viewpoint of increasing the flexibility of the film to be formed and improving washing durability, the component (B) is preferably one or more selected from the group consisting of polymethylsilsesquioxane, polypropylsilsesquioxane, (acrylates/ polytrimethylsiloxymethacrylate) copolymer, (acrylates/dimethicone) copolymer, (norbornene/tris(trimethylsiloxy)silylnorbornene) copolymer, vinylpyrrolidone/N,N-dimethylaminoethyl methacrylic acid copolymer diethyl sulfate, (acrylates/methoxy methacrylate PEG-23) copolymer, and (dimethylacrylamide/hydroxyethyl acrylate/methoxyethyl acrylate) copolymer, more preferably one or more selected from the group consisting of polypropylsilsesquioxane, (acrylates/dimethicone) copolymer, vinylpyrrolidone/N,N-dimethylaminoethyl methacrylic acid copolymer diethyl sulfate, (acrylates/methoxy methacrylate PEG-23) copolymer, and (dimethylacrylamide/hydroxyethyl acrylate/methoxyethyl acrylate) copolymer, and even preferably one or more selected from the group consisting of polypropylsilsesquioxane, (acrylates/dimethicone) copolymer, (acrylates/methoxy methacrylate PEG-23) copolymer, and (dimethylacrylamide/hydroxyethyl acrylate/methoxyethyl acrylate) copolymer. From the viewpoint of further improving washing durability, one or more selected from the group consisting of (acrylates/dimethicone) copolymer, (acrylates/methoxy methacrylate PEG-23) copolymer, and (dimethylacrylamide/hydroxyethyl acrylate/methoxyethyl acrylate) copolymer is preferable, and one or more selected from the group consisting of (acrylates/dimethicone) copolymer and (acrylates/methoxy methacrylate PEG-23) copolymer is more preferable. Further, polypropylsilsesquioxane is more preferred from the viewpoint of further improving ease of applying and spreading.

<Component (C): Volatile Solvent>

From the viewpoint of dissolving or dispersing the component (A), the component (B), and other components, from the viewpoint of improving washing durability, from the viewpoint of ease of applying and spreading the cosmetic composition, and from the viewpoint of imparting a smooth feel, the cosmetic composition of the present invention contains a volatile solvent as the component (C). The volatile solvent defined in the present invention does not include water.

The component (C) includes an alcohol-based solvent, an ether-based solvent, a ketone-based solvent, an ester-based solvent, a hydrocarbon-based solvent, and a silicone-based solvent, all of which have the volatility defined above.

The alcohol-based solvent includes ethanol, 1-propanol, 2-propanol, 1-butanol, 2-butanol, and benzyl alcohol.

The ether-based solvent includes diethyl ether and tetrahydrofuran, and the ketone-based solvent includes acetone and methyl ethyl ketone.

The ester-based solvent includes methyl acetate, ethyl acetate, butyl acetate, and isobutyl acetate.

The hydrocarbon-based solvent includes light liquid isoparaffin (containing, as a main component, isoparaffin having 8 to 16 carbon atoms), pentane, isopentane, hexane, isohexene, heptane, isoheptane, decane, isodecane, dodecane, isododecane, tridecane, isotridecane, tetradecane, and isotetradecane.

The silicone-based solvent include dimethylpolysiloxane having a viscosity at 25°C of 10 $mm^2/s$ or less, alkyltrimethicone such as methyl trimethicone, and methylphenylpolysiloxane having a viscosity at 25°C of 20 $mm^2/s$ or less. One or two or more of these can be used as the component (C).

From the viewpoint of ease of applying and spreading the cosmetic composition and from the viewpoint of imparting a smooth feel, the component (C) is preferably one or more selected from the group consisting of an alcohol-based solvent, a hydrocarbon-based solvent, and a silicone-based solvent, more preferably one or more selected from the group consisting of ethanol, 1-propanol, 2-propanol, 1-butanol, 2-butanol, pentane, isopentane, hexane, isohexane, heptane, isoheptane, decane, isodecane, dodecane, isododecane, tridecane, isotridecane, tetradecane, isotetradecane, light liquid isoparaffin, dimethylpolysiloxane having a viscosity at 25°C of 10 $mm^2/s$ or less, methyl trimethicone, and methylphenylpolysiloxane having a viscosity at 25°C of 20 $mm^2/s$ or less, even preferably one or more selected from the group consisting of ethanol, isodecane, isododecane, isotetradecane, light liquid isoparaffin, dimethylpolysiloxane having a viscosity at 25°C of 5 $mm^2/s$ or less, and methyl trimethicone, and even more preferably one or more selected from the group consisting of ethanol, isodecane, isododecane, isotetradecane, and light liquid isoparaffin.

<Content>

The content of the component (A) in the cosmetic composition is, from the viewpoint of improving film formability and washing durability, and from the viewpoint of ease of applying and spreading the cosmetic composition, preferably 0.5% by mass or more, more preferably 1% by mass or more, even preferably 2% by mass or more, and is, from the viewpoint of improving washing durability, from the view point of ease of applying and spreading the cosmetic composition, and from the view point of imparting smoothness, preferably 30% by mass or less, more preferably 25% by mass or less, even preferably 20% by mass or less, even more preferably 18% by mass or less, still preferably 16% by mass or less, and still more preferably 14% by mass or less. A specific range of the content of the component (A) in the cosmetic composition of the present invention is preferably 0.5% by mass or more and 30% by mass or less, more preferably 0.5% by mass or more and 25% by mass or less, even preferably 1% by mass or more and 20% by mass or less, even more preferably 2% by mass or more and 18% by mass or less, still preferably 2% by mass or more and 16% by mass or less, and still more preferably 2% by mass or more and 14% by mass or less.

The content of the component (B) in the cosmetic composition is, from the viewpoint of increasing the flexibility of the film to be formed, and improving washing durability, from the viewpoint of ease of applying and spreading the cosmetic composition, and from the viewpoint of imparting smoothness, preferably 0.1% by mass or more, more preferably

0.5% by mass or more, even preferably 1% by mass or more, and even more preferably 2% by mass or more, and is, from the viewpoint of improving film formability and durability, as well as ease of applying and spreading the cosmetic composition, preferably 30% by mass or less, more preferably 25% by mass or less, even preferably 20% by mass or less, even more preferably 15% by mass or less, and still preferably 10% by mass or less. A specific range of the content of the component (B) in the cosmetic composition of the present invention is preferably 0.1% by mass or more and 30% by mass or less, more preferably 0.5% by mass or more and 25% by mass or less, even preferably 0.5% by mass or more and 20% by mass or less, even more preferably 1% by mass or more and 20% by mass or less, still preferably 1% by mass or more and 15% by mass or less, and still more preferably 2% by mass or more and 10% by mass or less.

The total content of the component (A) and the component (B) in the cosmetic composition of the present invention is, from the viewpoint of improving film formability and washing durability, preferably 0.6% by mass or more, more preferably 1% by mass or more, even preferably 2% by mass or more, even more preferably 3% by mass or more, still preferably 5% by mass or more, still more preferably 7% by mass or more, and further more preferably 8% by mass or more. Moreover, from the viewpoint of ease of applying and spreading the cosmetic composition as well as imparting smoothness, preferably 40% by mass or less, more preferably 35% by mass or less, even preferably 30% by mass or less, even more preferably 25% by mass or less, still preferably 20% by mass or less, and still more preferably 15% by mass or less. A specific range of the total content of the component (A) and the component (B) in the cosmetic composition of the present invention is preferably 0.6% by mass or more and 40% by mass or less, more preferably 1% by mass or more and 40% by mass or less, even preferably 1% by mass or more and 35% by mass or less, even more preferably 2% by mass or more and 30% by mass or less, still preferably 2% by mass or more and 25% by mass or less, still more preferably 3% by mass or more and 20% by mass or less, further more preferably 3% by mass or more and 15% by mass or less, even further more preferably 5% by mass or more and 15% by mass or less, still further more preferably 7% by mass or more and 15% by mass or less, and still further more preferably 8% by mass or more and 15% by mass or less.

Moreover, the ratio of the content by mass of the component (A) to the total content by mass of the component (A) and the component (B) in the cosmetic composition of the present invention, [(A)/{(A)+(B)}] is, from the viewpoint of improving film formability and washing durability, preferably 10% or more, more preferably 15% or more, even preferably 20% or more, even more preferably 30% or more, and still preferably 40% or more. In addition, from the viewpoint of washing durability, ease of applying and spreading the cosmetic composition, and imparting smoothness, it is preferably 90% or less, more preferably 80% or less, even preferably 75% or less, even more preferably 70% or less, still preferably 65% or less, and still more preferably 60% or less. A specific range of the ratio of the content by mass of the component (A) to the total content by mass of the component (A) and the component (B) in the cosmetic composition of the present invention [(A)/{(A)+(B)}] is preferably 10% or more and 90% or less, more preferably 15% or more and 80% or less, even preferably 15% or more and 75% or less, even more preferably 20% or more and 70% or less, stillpreferably 20% or more and 65% or less, still more preferably 30% or more and 65% or less, and further more preferably 40% or more and 60% or less.

The content of the component (C) in the cosmetic composition of the present invention is, from the viewpoint of improving washing durability, from the viewpoint of ease of applying and spreading the cosmetic composition, and from the viewpoint of imparting a smooth feel, preferably 50% by mass or more, more preferably 60% by mass or more, even preferably 65% by mass or more, and even more preferably 70% by mass or more, and is, from the viewpoint of improving washing durability as well as ease of applying and spreading the cosmetic composition, preferably 99% by mass or less, more preferably 98% by mass or less, even preferably 95% by mass or less, and even more preferably 90% by mass or less. A specific range of the content of the component (C) in the cosmetic composition of the present invention is 50% by mass or more, preferably 50% by mass or more and 99% by mass or less, more preferably 60% by mass or more and 98% by mass or less, even preferably 65% by mass or more and 95% by mass or less, even more preferably 70% by mass or more and 90% by mass or less, and still preferably 70% by mass or more and 85% by mass or less.

The ratio of the content by mass of the component (A) to the content by mass of the component (C) in the cosmetic composition of the present invention, [(A)/(C)] is, from the viewpoint of improving film formability and washing durability, preferably 0.3% or more, more preferably 0.5% or more, even preferably 1% or more, and even more preferably 2% or more, and from the viewpoint of improving washing durability and from the view point of ease of applying and spreading the cosmetic composition, as well as imparting smoothness, it is preferably 60% or less, more preferably 50% or less, even preferably 40% or less, even more preferably 30% or less, and still preferably 25% or less. A specific range of the ratio of the content by mass of the component (A) to the content by mass of the component (C) in the cosmetic composition of the present invention, [(A)/(C)] is, preferably 0.3% or more and 60% or less, more preferably 0.5% or more and 50% or less, even preferably 1% or more and 40% or less, even more preferably 2% or more and 30% or less, and still preferably 2% or more and 25% or less.

The ratio of the content by mass of the component (B) to the content by mass of the component (C) in the cosmetic composition of the present invention, [(B)/(C)] is, from the viewpoint of improving film formability and washing

durability, preferably 0.2% or more, more preferably 0.5% or more, even preferably 1% or more, and even more preferably 2% or more, and from the viewpoint of improving film formability and durability, as well as ease of applying and spreading the cosmetic composition, it is preferably 60% or less, more preferably 50% or less, even preferably 40% or less, even more preferably 30% or less, and still preferably 20% or less. A specific range of the ratio of the content by mass of the component (B) to the content by mass of the component (C) in the cosmetic composition of the present invention, [(B)/(C)] is, preferably 0.2% or more and 60% or less, more preferably 0.5% or more and 50% or less, even preferably 1% or more and 40% or less, even more preferably 2% or more and 30% or less, and still preferably 2% or more and 20% or less.

The ratio of the total content by mass of the component (A) and the component (B) to the content by mass of the component (C) in the cosmetic composition of the present invention, [{ (A)+(B)}/(C)] is, from the viewpoint of improving film formability and washing durability, preferably 1.2% or more, more preferably 2% or more, even preferably 3% or more, even more preferably 5% or more, and still preferably 10% or more. In addition, from the viewpoint of improving film formability and durability, as well as ease of applying and spreading the cosmetic composition, it is preferably 95% or less, more preferably 90% or less, even preferably 80% or less, even more preferably 60% or less, still preferably 50% or less, and still more preferably 30% or less. A specific range of the ratio of the total content by mass of the component (A) and the component (B) to the content by mass of the component (C) in the cosmetic composition of the present invention, [{ (A)+(B)}/(C)] is, preferably 1.2% or more and 95% or less, more preferably 2% or more and 90% or less, even preferably 3% or more and 80% or less, even more preferably 3% or more and 60% or less, still preferably 5% or more and 50% or less, and still more preferably 10% or more and 30% or less.

<Component (D): Functional Powder>

The cosmetic composition of the present invention can further contain a functional powder as a component (D), depending on the product form thereof.

In the present invention, the functional powder means a powder capable of providing various characteristics such as coloring performance, concealing performance, gloss, UV scattering, and feel controlling. In the case where the cosmetic composition of the present invention is a sunscreen cosmetic material, preferably, a UV scattering agent is incorporated therein as the component (D), from the viewpoint of providing a desired sunscreen effect. In the case where the cosmetic composition of the present invention is a makeup cosmetic composition or a hair dye composition, preferably, a pigment is incorporated therein as the component (D), from the viewpoint of providing a desired color tone.

As the UV scattering agent, preferably usable is one or more metal oxide powder selected from the group consisting of zinc oxide, titanium oxide and cerium oxide. An average particle size of the fine particle metal oxide powder is, from the viewpoint of UV protective effect, preferably 10 to 500 nm, more preferably 12 to 100 nm, and even preferably 15 to 50 nm. The average particle size can be measured according to a laser diffraction/scattering method.

The pigment may be any pigment generally used in makeup cosmetic materials, hair dye compositions, and the like, and examples thereof include a white inorganic pigment such as titanium oxide, zinc oxide, cerium oxide and barium sulfate; a colored inorganic pigment such as yellow iron oxide, black iron oxide, red iron oxide, carbon black, chromium oxide, chromium hydroxide, Prussian blue and ultramarine blue; a luster powder such as titanium oxide-coated mica, titanium oxide-coated bismuth oxychloride, iron oxide-coated mica titanium, iron oxide mica, Prussian blue-processed mica titanium, carmine-processed mica titanium, bismuth oxychloride, and fish scale guanine; an organic pigment such as Red No. 201, Red No. 202, Red No. 205, Red No. 226, Red No. 228, Orange No. 203, Orange No. 204, Blue No. 404, and Yellow No. 401; a chelate pigment such as a zirconium, barium or aluminum chelate of Red No. 3, Red No. 104, Red No. 106, Orange No. 205, Yellow No. 4, Yellow No. 5, Green No. 3 or Blue No. 1; and a composite pigment such as fine particle titanium oxide-coated mica titanium, fine particle zinc oxide-coated mica titanium, barium sulfate-coated mica titanium, titanium oxide-containing silicon dioxide and zinc oxide-containing silicon dioxide. One or two or more of these can be used. Those prepared by coating the surfaces of these functional powders with various surface treatment agents are also usable as pigment. The surface treatment is not specifically limited. Various surface treatments can be applied to the powders, and examples thereof include fluorine compound treatment, silicone treatment, silicone resin treatment, pendant treatment, silane coupling agent treatment, titanium coupling agent treatment, oil treatment, metal soap treatment, N-acylated lysine treatment, polyethylene glycol treatment, PVA treatment, polyacrylic acid treatment, hyaluronic acid treatment, alginic acid treatment, inorganic compound treatment, plasma treatment and mechanochemical treatment, and the powders can be previously surface-treated by any of these treatments.

In the case where the cosmetic composition of the present invention contains the component (D), the content thereof is, from the viewpoint of providing desired performance, preferably 0.01% by mass or more in the cosmetic composition, more preferably 0.1% by mass or more, and even preferably 0.5% by mass or more, and is, from the viewpoint of dispersibility in the cosmetic composition and economic efficiency, and from the viewpoint of maintaining a good feel, preferably 30% by mass or less, more preferably 20% by mass or less, and even preferably 15% by mass or less. A specific range of the content of the component (D) in the cosmetic composition of the present invention is preferably 0.01% by mass or more and 30% by mass or less, more preferably 0.1% by mass or more and 20% by mass

or less, and even preferably 0.5% by mass or more and 15% by mass or less.

<Water>

The cosmetic composition of the present invention preferably has a low water content from the viewpoint of solubility of the component (A) and the component (B). The content thereof in the cosmetic composition is preferably 10% by mass or less, more preferably 5% by mass or less, even preferably less than 5% by mass, even more preferably less than 2% by mass, and still preferably substantially 0% by mass.

<Solid Oil>

From the viewpoint of ease of applying and spreading the cosmetic composition, from the viewpoint of improving film formability and washing durability, and from the viewpoint of imparting smoothness, the cosmetic composition of the present invention preferably has a low content of solid oil. The solid oil is an oil that is solid at 25°C, and includes a paraffin wax such as solid paraffin, a polyolefin wax such as polyethylene wax, and beeswax. The content of the solid oil in the cosmetic composition is preferably less than 50% by mass, more preferably less than 20% by mass, even preferably less than 10% by mass, even more preferably less than 5% by mass, and still preferably less than 1% by mass.

<Volatile Cyclic Silicone>

From the viewpoint of increasing the drying speed after application, the content of a volatile cyclic silicone such as decamethylcyclopentasiloxane in the cosmetic composition of the present invention is preferably small. This is because a volatile silicone oil takes a longer time for evaporation than a volatile hydrocarbon oil, and tends to prolong the time to be taken until drying after application to skin or hair. The content of a volatile cyclic silicone in the cosmetic composition is preferably less than 5% by mass in the cosmetic composition, more preferably less than 2% by mass, even preferably less than 1% by mass, even more preferably less than 0.5% by mass, still preferably less than 0.1% by mass, and still more preferably 0% by mass.

<Silicone-based Surfactant>

The cosmetic composition of the present invention preferably has a low content of silicone-based surfactant from the viewpoint of improving film formability and washing durability. The silicone-based surfactant refers to a surfactant having a silicone structure, typically a polysiloxane structure, and may have a hydrophilic group, hydrophilic polymer chain, etc. in a side chain, terminal, etc. The content of the silicone-based surfactant in the cosmetic composition is preferably less than 5% by mass, more preferably less than 2% by mass, even preferably less than 1% by mass, even more preferably less than 0.5% by mass, and still preferably less than 0.1% by mass.

<Oil Gelling Agent>

The cosmetic composition of the present invention preferably contains a low content of oil gelling agent such as fatty acid dextrin, hydrophobic silica, and lipophilic clay mineral, from the viewpoint of improving film formability and washing durability, and from the view point of imparting smoothness. The content of the oil gelling agent in the cosmetic composition is preferably less than 5% by mass, more preferably less than 2% by mass, even preferably less than 1% by mass, even more preferably less than 0.5% by mass, still preferably less than 0.1% by mass, and still more preferably substantially 0% by mass.

<Non-volatile Liquid Oil Agent>

The cosmetic composition of the present invention preferably has a low content of non-volatile liquid oil agent from the viewpoint of improving film formability and washing durability, and from the viewpoint of imparting smoothness. The content of the non-volatile liquid oil agent in the cosmetic composition is preferably less than 50% by mass, more preferably less than 40% by mass, even preferably less than 30% by mass, even more preferably less than 20% by mass, and still preferably less than 10% by mass.

<Polyhydric Alcohol>

The cosmetic composition of the present invention preferably has a low content of polyhydric alcohol, from the viewpoint of increasing the drying speed after application, from the view point of improving film formability and washing

durability, and from the view point of imparting smoothness. Examples of the polyhydric alcohol include propylene glycol and glycerin. The content of the polyhydric alcohol in the cosmetic composition is preferably less than 5% by mass, more preferably less than 2% by mass, even preferably less than 1% by mass, even more preferably less than 0.5% by mass, and still preferably less than 0.1% by mass.

<Other Components>

The cosmetic composition of the present invention can contain components generally used in cosmetic compositions, for example, an antioxidant, a fragrance, a colorant, a dye, a preservative, a thickener, a pH regulator, a blood circulation promoter, a cooling sensation agent, an antiperspirant, a bactericide, a skin activator, a moisturizer and a refrigerant, in addition to the above-mentioned components.

The cosmetic composition of the present invention can be produced according to an ordinary method.

<Formulation Form, etc.>

The formulation form of the cosmetic composition of the present invention is not specifically limited, and depending on the product form thereof, the cosmetic composition can have various formation forms such as liquid, paste, cream, gel, foam, spray and wax.

The cosmetic composition of the present invention may be in a form of an emulsified composition such as an oil-in-water type and a water-in-oil type, or may be in a form of a non-emulsified composition. However, a non-emulsified composition is preferable from the viewpoint of increasing the drying speed after application, from the view point of improving film formability and washing durability, and from the viewpoint of imparting smoothness.

The cosmetic composition of the present invention is more preferably a waterless composition. Here, the waterless composition means a composition having a water content of less than 1% by mass, preferably less than 0.5% by mass, and more preferably less than 0.1% by mass.

The cosmetic composition of the present invention includes various skin cosmetic compositions, eyebrow or eyelash makeup compositions and hair cosmetic compositions.

The skin cosmetic composition includes various skin cosmetic compositions for makeup, foundation, skincare, sunscreen.

The eyebrow or eyelash makeup composition includes various eyebrow or eyelash makeup compositions such as mascara, mascara base coat, mascara topcoat, and eyebrow mascara.

The hair cosmetic composition includes a hair wash composition such as shampoo, as well as a rinse composition, a conditioner composition, a treatment composition (including nonwashing type), a styling composition, a hair dye composition, and a hair tonic composition. Among these, from the viewpoint of the effectiveness of the advantageous effects of the present invention, preferred is a rinse composition, a conditioner composition, a treatment composition, a styling composition, or a hair dye composition.

Among the above, the cosmetic composition of the present invention is preferably a skin cosmetic composition or a hair cosmetic composition, more preferably a hair cosmetic composition, even preferably a rinse composition, a conditioner composition, a treatment composition, or a hair dye composition, and even more preferably a hair dye composition, from the viewpoint of exhibiting the effect of applying and spreading over a wide area.

The above-mentioned cosmetic composition is preferably a so-called leave-on preparation that is used without washing after application to keratin substances such as skin, eyebrow, eyelash and hair.

[Cosmetic Kit]

The present invention provides a cosmetic kit provided with two or more compositions, wherein the following components (A) to (C) are contained in a cosmetic composition obtained by mixing the compositions:

a component (A): a film-forming polymer containing an M unit represented by $(R^1)_3SiO_{1/2}$ and a Q unit represented by $SiO_{4/2}$ (wherein $R^1$ represents a hydrocarbon group having 1 or more and 12 or less carbon atoms and optionally substituted with fluorine, or a hydroxy group, and plural $R^1$'s can be the same as or different from each other),
a component (B): a film-forming polymer other than the component (A), and
a component (C): a volatile solvent.

The cosmetic kit of the present invention includes two or more compositions, and the compositions are mixed before use. More specifically, the cosmetic kit of the present invention can be used by mixing two or more compositions provided in the cosmetic kit before use to prepare a cosmetic composition containing the components (A) to (C), and applying the cosmetic composition to keratin substances such as skin, eyebrow, eyelash, and hair by coating. The

components (A) to (C) may be contained, among compositions provided in the cosmetic kit, in any one of the compositions constituting the cosmetic composition.

The content of the component (C) in the cosmetic composition obtained by mixing two or more compositions provided in the cosmetic kit is preferably 50% by mass or more, more preferably 60% by mass or more, even preferably 70% by mass or more, and even more preferably 80% by mass or more, and is preferably 99% by mass or less, more preferably 98% by mass or less, and even preferably 95% by mass or less.

The components (A) to (C) used in the cosmetic kit and preferred embodiments thereof are the same as those in the cosmetic composition described hereinabove. Moreover, each composition provided in the cosmetic kit may contain other components exemplified in the cosmetic composition as necessary.

The cosmetic kit of the present invention may further include a composition that does not contain any of the components (A) to (C). Moreover, the cosmetic kit of the present invention may further include a composition that does not constitute the cosmetic composition. For example, it may be a multi-formulation cosmetic kit including a composition 1, a composition 2, and a composition 3, wherein a cosmetic composition containing the components (A) to (C) obtained by mixing the compositions 1 and 2 is a first formulation and the composition 3 is a second formulation.

[Treatment Method for Keratin Substances]

The present invention also provides a treatment method for keratin substances, including a step of applying the cosmetic composition to a keratin substance and then drying it.

The keratin substance includes skin, eyebrow, eyelash, hair and nails, and is preferably skin, eyelash, eyebrow or hair, and more preferably hair. The keratin substance to which the cosmetic composition is applied may be in any of a dry state or wet state, but from the viewpoint of attaining the effects of the present invention, the cosmetic composition is preferably applied to a keratin substance in a dry state.

From the viewpoint of uniform application, and from the viewpoint of improving the uniformity of the structure of the film to be formed, it is preferable that the cosmetic composition of the present invention is temporarily compatibilized or dispersed prior to application to keratin substances, and then applied to the surfaces of keratin substances. As the temporarily compatibilizing or dispersing method, arbitrarily employable is any of a thermodynamical method of heating, a physical method of mechanically imparting shear stress, or a chemical method of adding a compatible solvent, etc. From the viewpoint of userfriendliness, preferably, the cosmetic composition is uniformly compatibilized or dispersed by a physical method of stirring or shaking, etc.

In the case where the cosmetic composition of the present invention is a skin cosmetic composition, or an eyebrow or eyelash cosmetic composition, preferably, the composition is applied to skin, eyebrow or eyelash and then spontaneously dried. From the viewpoint of maintaining various effects of the cosmetic composition, it is preferable that the composition is not washed off after drying, and is used as a leave-on preparation.

The skin cosmetic composition and the eyebrow or eyelash cosmetic composition is, after applied to skin, eyebrow or eyelash, preferably dried before being brought into contact with clothes and other articles. The drying time is not specifically limited so far as, after the cosmetic composition has been applied, it can substantially form a film on the surface of skin, eyebrow or eyelash, and the time can be appropriately controlled depending on the coating amount and the coating area, and preferably, the formed film is dried for 4 minutes or less, and more preferably 2 minutes or less.

In the case where the cosmetic composition of the present invention is a hair cosmetic composition, from the viewpoint of promptly forming a film, the hair treatment method preferably includes a step of applying the hair cosmetic composition to hair and then drying it.

From the viewpoint of maintaining various effects of the hair cosmetic composition, it is preferable that the composition is applied to hair in a dry state and then dried, and is used as a leave-on preparation that is not washed off after application. Drying the hair after application of the hair cosmetic composition thereto may be spontaneous drying, or the hair may be dried using a device such as a hair drier hood, a hand hair drier, or a straight iron.

In the case of using the device, preferably, the hair is dried at a temperature of 40 to 220°C from the viewpoint of suppressing thermal damages of keratin substances. More preferred is drying with a hair drier hood or a hand hair drier, and the drying temperature is preferably 40 to 110°C, and more preferably 50 to 90°C.

The drying time is not specifically limited so far as a film is substantially formed on the surface of hair, and can be appropriately controlled depending on the amount and the quality of hair. For example, the time may fall within a range of 10 seconds to 120 minutes.

After drying, the hair may be brushed to be straggly.

In the treatment method of the present invention, the amount of the cosmetic composition to be applied to keratin substances is not specifically limited. In the case of a skin cosmetic composition, in general, the amount falls within a range of 0.1 to 1000 mg per $cm^2$ of skin. In the case of an eyebrow or eyelash cosmetic composition or a hair cosmetic composition, in general, the amount falls within a range of 0.005 to 1 g per gram of eyebrow, eyelash or hair.

[Hair Dyeing Method]

The present invention further provides a hair dyeing method that includes a step of applying the hair dye composition to hair and then drying it.
The hair dye composition is applied to hair and then dried, and is used without washing. Drying the hair after applying the hair dye composition thereto may be spontaneous drying, or the hair may be dried with a hair drier or the like.
By the above-mentioned simple operation, the present invention can temporarily or semi-permanently dye hair as an out-bath treatment. In addition, the present invention can impart a smooth feel to hair and secure good color duration without discoloration by shampooing.
Regarding the above-mentioned embodiments, the present invention discloses the following.

<1> A cosmetic composition containing

a component (A): a film-forming polymer containing an M unit represented by $(R^1)_3SiO_{1/2}$ and a Q unit represented by $SiO_{4/2}$ (wherein $R^1$ represents a hydrocarbon group having 1 or more and 12 or less carbon atoms and optionally substituted with fluorine, or a hydroxy group, and plural $R^1$'s can be the same as or different from each other),
a component (B): a film-forming polymer other than the component (A), and
a component (C): a volatile solvent,

wherein the component (C) has a content of 50% by mass or more.
<2> A cosmetic composition containing

a component (A): a film-forming polymer containing an M unit represented by $(R^1)_3SiO_{1/2}$ and a Q unit represented by $SiO_{4/2}$ (wherein $R^1$ represents a hydrocarbon group having 1 or more and 12 or less carbon atoms and optionally substituted with fluorine, or a hydroxy group, and plural $R^1$'s can be the same as or different from each other),
a component (B): a film-forming polymer other than the component (A), and
a component (C): a volatile solvent,

wherein the component (C) has a content of 70% by mass or more and 90% by mass or less, and
a ratio of a content by mass of the component (A) to a total content by mass of the component (A) and the component (B), [(A)/{(A) + (B)}] is, 15% or more and 75% or less.

<3> A cosmetic composition containing

a component (A): a film-forming polymer containing an M unit represented by $(R^1)_3SiO_{1/2}$ and a Q unit represented by $SiO_{4/2}$ (wherein $R^1$ represents a hydrocarbon group having 1 or more and 12 or less carbon atoms and optionally substituted with fluorine, or a hydroxy group, and plural $R^1$'s can be the same as or different from each other),
a component (B): a film-forming polymer other than the component (A), and
a component (C): a volatile solvent,

wherein the component (C) has a content of 70% by mass or more and 90% by mass or less, and
a ratio of a content by mass of the component (A) to a total content by mass of the component (A) and the component (B), [(A)/{(A) + (B)}] is, 30% or more and 65% or less.

<4> The cosmetic composition according to any one of <1> to <3>, wherein $R^1$ in the component (A) is preferably an alkyl group having 1 or more and 12 or less carbon atoms and optionally substituted with fluorine, an aryl group having 6 or more and 12 or less carbon atoms, or an aralkyl group having 7 or more and 12 or less carbon atoms, more preferably an alkyl group having 1 or more and 8 or less carbon atoms and optionally substituted with fluorine, or a phenyl group, even preferably an alkyl group having 1 or more and 6 or less carbon atoms and optionally substituted with fluorine, or a phenyl group, even more preferably a trifluoropropyl group, an alkyl group having 1 or more and 4 or less carbon atoms, or a phenyl group, still preferably a trifluoropropyl group, a methyl group, an ethyl group, an n-propyl group, an isopropyl group, or an n-butyl group, still more preferably a trifluoropropyl group, a methyl group or an n-propyl group, and further more preferably a methyl group.
<5> The cosmetic composition according to any one of <1> to <4>, wherein the component (A) is one or more selected from the group consisting of trimethylsiloxysilicate, phenylpropyldimethylsiloxysilicate, fluorine-modified

alkyldimethylsiloxysilicates, and crosspolymers produced by crosslinking these siloxysilicates with dimethiconol, preferably one or more selected from the group consisting of trimethylsiloxysilicate, trifluoropropyldimethyltrimethylsiloxysilicate and (trimethylsiloxysilicate/dimethiconol) crosspolymer, more preferably one or more selected from trimethylsiloxysilicate, and trifluoropropyldimethyltrimethylsiloxysilicate, and even preferably trimethylsiloxysilicate.
<6> The cosmetic composition according to any one of <1> to <5>, wherein the component (B) is a hydrophobic film-forming polymer other than the component (A), preferably one or more selected from the group consisting of the following components (B 1) to (B6), more preferably one or more selected from the group consisting of the component (B 1), the component (B2) and the component (B6), even preferably one or more selected from the group consisting of the component (B 1) and the component (B2), and even more preferably the component (B2):

(B 1) a silicone resin containing a T unit represented by $R^1SiO_{3/2}$ and substantially not containing a Q unit represented by $SiO_{4/2}$ ($R^1$ is the same as above)
(B2) an acrylic silicone polymer
(B3) a silicone-modified alicyclic structure-containing polymer
(B4) a silicone-modified pullulan
(B5) a polyurea/urethane silicone
(B6) a non-silicone-based polymer with a glass transition temperature of 200°C or lower.

<7> The cosmetic composition according to <6>, wherein the component (B 1) is a silicone resin containing one or more units selected from the group consisting of an M unit represented by $(R^1)_3SiO_{1/2}$ and a D unit represented by $(R^1)_2SiO_{2/2}$, and is preferably a silicone resin represented by $[R^1SiO_{3/2}]_a[(R^1)_3SiO_{1/2}]_b$ (a and b each are an average repeating unit number and a>0 and b≥0) containing a T unit and optionally containing an M unit.
<8> The cosmetic composition according to <7>, wherein $R^1$ in the component (B1) is preferably an alkyl group having 1 or more and 4 or less carbon atoms, or a phenyl group, more preferably a methyl group, an ethyl group, an n-propyl group, or an isopropyl group, and even preferably a methyl group, an n-propyl group, or an isopropyl group.
<9> The cosmetic composition according to any one of <6> to <8>, wherein the component (B1) is a polysilsesquioxane, preferably one or more selected from the group consisting of polymethylsilsesquioxane, polypropylsilsesquioxane, polyphenylsilsesquioxane, polymethylphenylsilsesquioxane, and fluorine-modified alkyldimethylpolysilsesquioxane, more preferably one or more selected from the group consisting of polymethylsilsesquioxane and polypropylsilsesquioxane, and even preferably polypropylsilsesquioxane.
<10> The cosmetic composition according to any one of <6> to <9>, wherein the component (B2) is one or more selected from the group consisting of an acrylic polymer having a carbosiloxane dendrimer structure in the side chain, an acryl-silicone graft copolymer, and a graft-type copolymer or alternate block-type copolymer where a structural unit of a polysiloxane group and a structural unit of a polymer of an unsaturated monomer bond via a sulfide bond, preferably one or more selected from the group consisting of an acrylic polymer having a carbosiloxane dendrimer structure in the side chain and an acryl-silicone graft copolymer, and more preferably (acrylates/dimethicone) copolymer.
<11> The cosmetic composition according to any one of <6> to <10>, wherein the component (B3) is a silicone-modified cyclic polyolefin, and preferably a silicone-modified polynorbornene represented by the following general formula (B3-1):

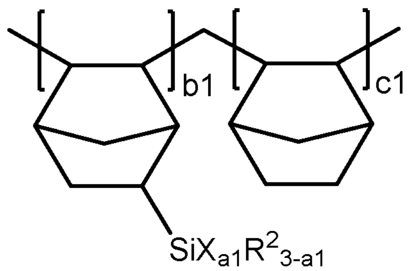

(B3-1)

wherein $R^2$ each independently represents an alkyl group having 1 or more and 12 or less carbon atoms, X represents a group represented by the following formula (i), a1 is an integer of 1 or more and 3 or less, b1 and c1 each are a repeating unit number, and are each independently an integer of 1 or more,

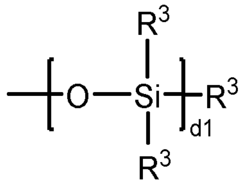

(i)

wherein $R^3$ each independently represents a hydrocarbon group having 1 or more and 12 or less carbon atoms, and d1 is an integer of 1 or more and 5 or less.

<12> The cosmetic composition according to any one of <6> to <11>, wherein the component (B4) is a pullulan having a silicone structure in the side chain, and preferably a silicone-modified pullulan in which at least a part of the hydrogen atoms of the OH groups in pullulan are substituted with a group represented by the following general formula (ii):

$$-R^4-SiX_{a1}R^2_{3-a1} \qquad (II)$$

wherein $R^4$ represents a single bond or a divalent organic group, $R^2$, X and a1 are the same as above, X is preferably a trimethylsiloxyl group, and a1 is preferably 3.

<13> The cosmetic composition according to any one of <6> to <12>, wherein the component (B5) is a polysiloxane/polyurea/polyurethane block terpolymer.

<14> The cosmetic composition according to any one of <6> to <13>, wherein the component (B6) is a film-forming polymer that has a glass transition temperature of 200°C or lower, preferably 150°C or lower, more preferably 100°C or lower, even preferably 60°C or lower, and even more preferably 5°C or lower, and does not contain a silicone structure.

<15> The cosmetic composition according to any one of <1> to <14>, wherein the component (B) is one or more selected from the group consisting of polymethylsilsesquioxane, polypropylsilsesquioxane, (acrylates/ polytrimethylsiloxymethacrylate) copolymer, (acrylates/dimethicone) copolymer, (norbornene/tris(trimethylsiloxy)silylnorbornene) copolymer, vinylpyrrolidone/N,N-dimethylaminoethyl methacrylic acid copolymer diethyl sulfate, (acrylates/methoxy methacrylate PEG-23) copolymer, and (dimethylacrylamide/hydroxyethyl acrylate/methoxyethyl acrylate) copolymer, preferably one or more selected from the group consisting of polypropylsilsesquioxane, (acrylates/dimethicone) copolymer, vinylpyrrolidone/N,N-dimethylaminoethyl methacrylic acid copolymer diethyl sulfate, (acrylates/methoxy methacrylate PEG-23) copolymer, and (dimethylacrylamide/hydroxyethyl acrylate/methoxyethyl acrylate) copolymer, more preferably one or more selected from the group consisting of polypropylsilsesquioxane, (acrylates/dimethicone) copolymer, (acrylates/methoxy methacrylate PEG-23) copolymer, and (dimethylacrylamide/hydroxyethyl acrylate/methoxyethyl acrylate) copolymer, even preferably one or more selected from the group consisting of (acrylates/dimethicone) copolymer, (acrylates/methoxy methacrylate PEG-23) copolymer, and (dimethylacrylamide/hydroxyethyl acrylate/methoxyethyl acrylate) copolymer, even more preferably one or more selected from the group consisting of (acrylates/dimethicone) copolymer and (acrylates/methoxy methacrylate PEG-23) copolymer, and still preferably (acrylates/dimethicone) copolymer.

<16> The cosmetic composition according to any one of <1> to <15>, wherein the component (C) is one or more selected from the group consisting of an alcohol-based solvent, an ether-based solvent, a ketone-based solvent, an ester-based solvent, a hydrocarbon-based solvent, and a silicone-based solvent having a boiling point of 260°C or lower under normal pressure, and preferably one or more selected from the group consisting of an alcohol-based solvent, a hydrocarbon-based solvent, and a silicone-based solvent.

<17> The cosmetic composition according to <16>, wherein the alcohol-based solvent is one or more selected from the group consisting of ethanol, 1-propanol, 2-propanol, 1-butanol, 2-butanol, and benzyl alcohol.

<18> The cosmetic composition according to <16> or <17>, wherein the hydrocarbon-based solvent is one or more selected from the group consisting of light liquid isoparaffin, pentane, isopentane, hexane, isohexene, heptane, isoheptane, decane, isodecane, dodecane, isododecane, tridecane, isotridecane, tetradecane, and isotetradecane.

<19> The cosmetic composition according to any one of <16> to <18>, wherein the silicone-based solvent is one or more selected from the group consisting of dimethylpolysiloxane having a viscosity at 25°C of 10 $mm^2/s$ or less, alkyltrimethicone, and methylphenylpolysiloxane having a viscosity at 25°C of 20 $mm^2/s$ or less.

<20> The cosmetic composition according to any one of <1> to <19>, wherein the component (C) is one or more selected from the group consisting of ethanol, 1-propanol, 2-propanol, 1-butanol, 2-butanol, pentane, isopentane, hexane, isohexane, heptane, isoheptane, decane, isodecane, dodecane, isododecane, tridecane, isotridecane, tetradecane, isotetradecane, light liquid isoparaffin, dimethylpolysiloxane having a viscosity at 25°C of 10 $mm^2/s$ or less, methyl trimethicone, and methylphenylpolysiloxane having a viscosity at 25°C of 20 $mm^2/s$ or less, preferably one or more selected from the group consisting of ethanol, isodecane, isododecane, isotetradecane, light liquid

isoparaffin, dimethylpolysiloxane having a viscosity at 25°C of 5 $mm^2/s$ or less, and methyl trimethicone, and more preferably one or more selected from the group consisting of ethanol, isodecane, isododecane, isotetradecane, and light liquid isoparaffin.

<21> The cosmetic composition according to any one of <1> to <20>, wherein the content of the component (A) in the cosmetic composition is preferably 0.5% by mass or more and 30% by mass or less, more preferably 0.5% by mass or more and 25% by mass or less, even preferably 1% by mass or more and 20% by mass or less, even more preferably 2% by mass or more and 18% by mass or less, still preferably 2% by mass or more and 16% by mass or less, and still more preferably 2% by mass or more and 14% by mass or less.

<22> The cosmetic composition according to any one of <1> to <21>, wherein the content of the component (B) in the cosmetic composition is preferably 0.1% by mass or more and 30% by mass or less, more preferably 0.5% by mass or more and 25% by mass or less, even preferably 0.5% by mass or more and 20% by mass or less, even more preferably 1% by mass or more and 20% by mass or less, still preferably 1% by mass or more and 15% by mass or less, and still more preferably 2% by mass or more and 10% by mass or less.

<23> The cosmetic composition according to any one of <1> to <22>, wherein the total content of the component (A) and the component (B) in the cosmetic composition is preferably 0.6% by mass or more and 40% by mass or less, more preferably 1% by mass or more and 40% by mass or less, even preferably 1% by mass or more and 35% by mass or less, even more preferably 2% by mass or more and 30% by mass or less, still preferably 2% by mass or more and 25% by mass or less, still more preferably 3% by mass or more and 20% by mass or less, further more preferably 3% by mass or more and 15% by mass or less, even further more preferably 5% by mass or more and 15% by mass or less, still further more preferably 7% by mass or more and 15% by mass or less, and still further more preferably 8% by mass or more and 15% by mass or less.

<24> The cosmetic composition according to any one of <1> and <4> to <23>, wherein the ratio of the content by mass of the component (A) to the total content by mass of the component (A) and the component (B) in the cosmetic composition [(A)/{(A)+(B)}] is preferably 10% or more and 90% or less, more preferably 15% or more and 80% or less, even preferably 15% or more and 75% or less, even more preferably 20% or more and 70% or less, still preferably 20% or more and 65% or less, still more preferably 30% or more and 65% or less, and further more preferably 40% or more and 60% or less.

<25> The cosmetic composition according to any one of <1> and <4> to <24>, wherein the content of the component (C) in the cosmetic composition is preferably 50% by mass or more and 99% by mass or less, more preferably 60% by mass or more and 98% by mass or less, even preferably 65% by mass or more and 95% by mass or less, even more preferably 70% by mass or more and 90% by mass or less, and still preferably 70% by mass or more and 85% by mass or less.

<26> The cosmetic composition according to any one of <1> to <25>, further containing a functional powder, preferably a pigment, as a component (D).

<27> The cosmetic composition according to <26>, wherein the content of the component (D) in the cosmetic composition is preferably 0.01% by mass or more and 30% by mass or less, more preferably 0.1% by mass or more and 20% by mass or less, and even preferably 0.5% by mass or more and 15% by mass or less.

<28> The cosmetic composition according to any one of <1> to <27>, wherein the content of water in the cosmetic composition is preferably 10% by mass or less, more preferably 5% by mass or less, even preferably less than 5% by mass, even more preferably less than 2% by mass, and still preferably substantially 0% by mass.

<29> The cosmetic composition according to any one of <1> to <28>, wherein the content of a solid oil in the cosmetic composition is preferably less than 50% by mass, more preferably less than 20% by mass, even preferably less than 10% by mass, even more preferably less than 5% by mass, and still preferably less than 1% by mass.

<30> The cosmetic composition according to any one of <1> to <29>, wherein the content of a volatile cyclic silicone, preferably decamethylcyclopentasiloxane, in the cosmetic composition is preferably less than 5% by mass, more preferably less than 2% by mass, even preferably less than 1% by mass, even more preferably less than 0.5% by mass, still preferably less than 0.1% by mass, and still more preferably 0% by mass.

<31> The cosmetic composition according to any one of <1> to <30>, wherein the content of a silicone-based surfactant in the cosmetic composition is preferably less than 5% by mass, more preferably less than 2% by mass, even preferably less than 1% by mass, even more preferably less than 0.5% by mass, and still preferably less than 0.1% by mass.

<32> The cosmetic composition according to any one of <1> to <31>, wherein the content of an oil gelling agent in the cosmetic composition is preferably less than 5% by mass, more preferably less than 2% by mass, even preferably less than 1% by mass, even more preferably less than 0.5% by mass, still preferably less than 0.1% by mass, and still more preferably substantially 0% by mass.

<33> The cosmetic composition according to any one of <1> to <32>, wherein the content of a non-volatile liquid oil agent in the cosmetic composition is preferably less than 50% by mass, more preferably less than 40% by mass, even preferably less than 30% by mass, even more preferably less than 20% by mass, and still preferably less than

10% by mass.
<34> The cosmetic composition according to any one of <1> to <33>, wherein the content of a polyhydric alcohol in the cosmetic composition is preferably less than 5% by mass, more preferably less than 2% by mass, even preferably less than 1% by mass, even more preferably less than 0.5% by mass, and still preferably less than 0.1% by mass.
<35> The cosmetic composition according to any one of <1> to <34>, which is a non-emulsified composition.
<36> The cosmetic composition according to any one of <1> to <35>, which is a waterless composition having a water content of less than 1% by mass, preferably less than 0.5% by mass, and more preferably less than 0.1% by mass.
<37> The cosmetic composition according to any one of <1> to <36>, which is a leave-on preparation.
<38> A skin cosmetic composition or hair cosmetic composition composed of the cosmetic composition according to any one of <1> to <37>.
<39> A hair dye composition composed of the cosmetic composition according to any one of <1> to <37>.
<40> A method for treating a keratin substance, including a step of applying the cosmetic composition according to any one of <1> to <37> to a keratin substance and then drying it.
<41> A method for treating hair, including a step of applying the hair cosmetic composition according to <38> to hair and then drying it.
<42> A method for dyeing hair, including a step of applying the hair dye composition according to <39> to hair and then drying it.
<43> A cosmetic kit provided with two or more compositions,
wherein the following components (A) to (C) are contained in a cosmetic composition obtained by mixing the compositions:

a component (A): a film-forming polymer containing an M unit represented by $(R^1)_3SiO_{1/2}$ and a Q unit represented by $SiO_{4/2}$ (wherein $R^1$ represents a hydrocarbon group having 1 or more and 12 or less carbon atoms and optionally substituted with fluorine, or a hydroxy group, and plural $R^1$'s can be the same as or different from each other),
a component (B): a film-forming polymer other than the component (A), and
a component (C): a volatile solvent.

<44> The cosmetic kit according to <43>, which is used by mixing the two or more compositions provided in the cosmetic kit before use to prepare a cosmetic composition containing the components (A) to (C), and applying the cosmetic composition to a keratin substance.

Examples

Hereinunder the present invention is described with reference to Examples, but the present invention is not restricted to the range of Examples. In these Examples, various measurements and evaluations were carried out according to the following methods.

<Measurement of Glass Transition Temperature (Tg)>

The Tg of the non-silicone polymer of the component (B) was measured under the following conditions.

Equipment used: "DSC1 STARe System" manufactured by METTLER TOLEDO
Cell used: Aluminum 100μL
Sample amount: 25 to 50 mg
Temperature raising rate: 5°C /min

<Preparation of Hair Bundles for Evaluation>

Human gray hair (100%) bundles (by Beaulax Co., Ltd., length 10 cm, mass 1 g) were shampooed with a plain shampoo having a formulation mentioned below, then rinsed with warm water at 40°C, and fully dried to prepare hair bundles for evaluation.

(Plain Shampoo Formulation)

| Ingredient | (mass%) |
|---|---|
| Polyoxyethylene (2) lauryl ether sodium sulfate (*1) | 15.5 |
| Lauric acid diethanolamide (*2) | 1.5 |
| Tetrasodium edetate | 0.3 |
| Sodium benzoate | 1.43 |
| Pure water | balance |
| Total | 100.0 |

*1: 57.4% by mass as Emal 227 (by Kao Corporation, active ingredient 27% by mass)
*2: Aminon L-02 (by Kao Corporation)

<Ease of Applying and Spreading>

0.05 to 0.2 ml of the hair cosmetic composition of each Example was dropwise applied to a commercial PET film (Lumirror Film T60-A4-100 μm, manufactured by Toray Corporation) and spread thereon with a bar coater (No. 60, manufactured by As One). After sufficiently drying at room temperature, it was scanned with a multifunction device (RICOH MP C5504) to create a JPEG image, and the area of the part where the composition was spread was calculated using image processing software "Image J". From the amount of the composition dropped and the area of the spread area, the spread area per 0.1 ml was calculated using the following formula.

$$\text{Spread area per } 0.1\text{ml } [\text{cm}^2] = \text{Spread area } [\text{cm}^2] / \text{Dripping amount } [\text{ml}] \times 0.1$$

The above test was carried out two times, and an average value is shown in Table. When the spread area per 0.1 ml was less than 12 $cm^2$, it was determined as a failure. A spread area of 12 $cm^2$ or more means ease of applying and spreading, 20 $cm^2$ or more means more ease of applying and spreading, and 50 $cm^2$ or more means further more ease of applying and spreading.

<Feel (Smoothness)>

0.15 g of the hair cosmetic composition of each Example was applied to the hair bundle for evaluation, and then was dried for 2 minutes with a hair drier ("P2-D250" by Hitachi Limited, setting HIGH) by applying warm air from a position separated by 18 cm from the hair bundle for hair treatment. Expert panelists organoleptically evaluated the feel of the treated hair bundles according to the following criteria, and a total point of N = 3 was calculated.

5: Feels very smooth
4: Feels smooth
3: Feels somewhat smooth
2: Feels slightly unsmooth
1: Feels not smooth

A total point of N = 3 of 6 or less means that the texture is strongly creaky and feels not smooth (fail). A total point of 7 to 8 means that the texture is smooth but slightly creaky, giving a slightly good feel. A total point of 9 to 11 indicates a good feel, and a total point of 12 or more indicates an even better feel.

<Washing Durability>

0.15 g of the hair cosmetic composition of each Example was applied to the hair bundle for evaluation, and then this was dried for 2 minutes with a hair drier ("P2-D250", manufactured by Hitachi, Limited., setting HIGH) by applying warm air from a position separated by 18 cm from the hair bundle for hair treatment.

The treated hair bundle was analyzed with a color difference meter (CR-400 by Konica Minolta, Inc.) in a CIE color system (L*,a*,b*). Then, this was shampooed with the plain shampoo having the formulation mentioned above, rinsed with warm water at 40°C and dried. The process was repeated 3 times. After shampooing 3 times, the dried hair bundle was analyzed with the color difference meter in the same manner as above, and according to the following formulation, a ΔE duration rate [%] after shampooing 3 times was calculated. L*, a* and b* were measured at different 6 points on the hair bundle (each at 2 central points of each region obtained by equally dividing the hair bundle into three

in the length direction), and the found data were averaged to give an average value.

$\Delta E_1$* of hair bundle after treatment and before shampooing

$$\Delta E_1^* = \{(L_1^* - L_0^*)^2 + (a_1^* - a_0^*)^2 + (b_1^* - b_0^*)^2\}^{1/2}$$

$\Delta E_2$* of hair bundle after shampooing 3 times

$$\Delta E_2^* = \{(L_2^* - L_0^*)^2 + (a_2^* - a_0^*)^2 + (b_2^* - b_0^*)^2\}^{1/2}$$

$$\Delta E \text{ duration rate [\%]} = \Delta E_2^* / \Delta E_1^* \times 100$$

$L_0$*, $a_0$*, $b_0$*: found data of hair bundle before treatment
$L_1$*, $a_1$*, $b_1$*: found data of hair bundle after treatment and before shampooing
$L_2$*, $a_2$*, $b_2$*: found data of hair bundle after shampooing 3 times
ΔE duration rate > 50% means that the treated hair bundle is considered to have good shampooing resistance and to be excellent in color sustainability. Further, ΔE duration rate > 55% means better, ΔE duration rate > 70% means even better, and ΔE duration rate > 80% means further better. ΔE duration rate ≤ 50% was determined as a failure.

Examples 1 to 15, Comparative Examples 1 to 2 (Preparation and Evaluation of Hair Cosmetic Compositions)

Ingredients shown in Table 1 were blended according to the formulation described in each Table, and then mixed until uniform to prepare hair cosmetic compositions. The resultant hair cosmetic compositions were evaluated according to the above-mentioned methods. The results are shown in Table 1.
The blending amount (mass%) shown in Table is an active ingredient amount.

Table 1

| | | | Tg (°C) | Example | | | | | | | | | | | | | | | Comparative Example | |
|---|---|---|---|---|---|---|---|---|---|---|---|---|---|---|---|---|---|---|---|---|
| | | | | 1 | 2 | 3 | 4 | 5 | 6 | 7 | 8 | 9 | 10 | 11 | 12 | 13 | 14 | 15 | 1 | 2 |
| (A) | Trimethylsiloxysilicate | X-21-5595 *1 | | | 18.6 | 12.8 | 7 | | 2.4 | 4.8 | 8.4 | 9.6 | 7 | 7 | 7 | 7 | 7 | | | 12 |
| | | SR1000 *2 | | 27.3 | | | | 1.74 | | | | | | | | | | 13.4 | 33 | |
| (B) | (B2) (acrylates/dimethicone) copolymer | KP-550 *3 | - | 19.7 | 13.4 | 9.2 | 5 | 1.26 | 9.6 | 7.2 | 3.6 | 2.4 | | | | | 5 | 9.6 | 24 | 0 |
| | (B1) polypropylsilsesquioxane | 680 ID Fluid *4 | - | | | | | | | | | | 5 | | | | | | | |
| | (B6) (dimethylacrylamide/hydroxyethyl acrylate/methoxyethyl acrylate) copolymer | Plas Cize L-2700 *5 | - | | | | | | | | | | | 5 | | | | | | |
| | (B6) (acrylates/methoxy methacrylate PEG-23) copolymer | Plas Cize L-188K *6 | - | | | | | | | | | | | | 5 | | | | | |
| | (B6) polyquaternium-11 | Gafquat 734 *7 | 70 | | | | | | | | | | | | | 5 | | | | |
| (C) | Isododecane | Marukasol R *8 | | 50 | 65 | 75 | 85 | 94 | 85 | 85 | 85 | 85 | 85 | 4.67 | 4.67 | 4.67 | 88 | 60 | 40 | 85 |
| | Ethanol | | | | | | | | | | | | | 80.33 | 78.58 | 80.33 | | | | |
| | Water | | | | | | | | | | | | | | 1.75 | | | | | |
| (D) | Iron oxide | Black BL-100P *9 | | 3 | 3 | 3 | 3 | 3 | 3 | 3 | 3 | 3 | 3 | 3 | 3 | 3 | 0 | 17 | 3 | 3 |
| Total | | | | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 |
| Total content of (A) + (B) (% by mass) | | | | 47 | 32 | 22 | 12 | 3 | 12 | 12 | 12 | 12 | 12 | 12 | 12 | 12 | 12 | 23 | 57 | 12 |
| Content of (C) (% by mass) | | | | 50 | 65 | 75 | 85 | 94 | 85 | 85 | 85 | 85 | 85 | 85 | 83.25 | 85 | 88 | 60 | 40 | 85 |
| (A)/{(A)+(B)}(%) | | | | 58 | 58 | 58 | 58 | 58 | 20 | 40 | 70 | 80 | 58 | 58 | 58 | 58 | 58 | 58 | 58 | 100 |
| (A)/(C) (%) | | | | 55 | 29 | 17 | 8 | 2 | 3 | 6 | 10 | 11 | 8 | 8 | 8 | 8 | 8 | 22 | 83 | 14 |
| (B)/(C) (%) | | | | 39 | 21 | 12 | 6 | 1 | 11 | 8 | 4 | 3 | 6 | 6 | 6 | 6 | 6 | 16 | 60 | - |
| {(A)+(B)}/(C) (%) | | | | 94 | 49 | 29 | 14 | 3 | 14 | 14 | 14 | 14 | 14 | 14 | 14 | 14 | 14 | 38 | 143 | 14 |
| Evaluation results | Ease of applying and spreading | Spread area per 0.1ml [$cm^2$] (average of N = 2) | | 13.00 | 24.73 | 66.14 | 57.86 | 49.00 | 77.88 | 51.94 | 84.39 | 88.12 | 70.09 | 50.35 | 45.20 | 14.42 | 78.11 | 23.27 | 10.76 | 86.34 |
| | Feel (smoothness) | Organoleptic evaluation (total point of N = 3) | | 10 | 10 | 12 | 12 | 14 | 13 | 12 | 9 | 8 | 7 | 7 | 7 | 7 | 12 | 9 | 6 | 5 |
| | Washing durability | ΔE duration rate after shampooing 3 times [%] | | 83.4 | 99.7 | 96.5 | 96.1 | 74.4 | 87.3 | 99.5 | 75.5 | 54.6 | 63.6 | 84.6 | 96.4 | 67.4 | 90.5 | 59.3 | 75.3 | 39.4 |

Ingredients shown in the table are as described below.

*1: X-21-5595, by Shin-Etsu Chemical Industry Co., Ltd., isododecane solution of trimethylsiloxysilicate (60 mass%)
*2: SR1000, by Momentive Performance Materials Japan LLC, trimethylsiloxysilicate
*3: KP-550, by Shin-Etsu Chemical Industry Co., Ltd., isododecane solution of (acrylates/dimethicone) copolymer (40 mass%)
*4: DOWSIL 680 ID Fluid, by Dow Toray Corporation, isododecane solution of polypropylsilsesquioxane (75 mass%)
*5: Plas Cize L-2700, by Goo Chemical Co., Ltd., ethanol solution of (dimethylacrylamide/hydroxyethyl acrylate/methoxyethyl acrylate) copolymer (70 mass%)
*6: Plas Cize L-188K, by Goo Chemical Co., Ltd., ethanol/aqueous solution of (acrylates/methoxy methacrylate PEG-23) copolymer (40 mass%) (ethanol 46%, water 14%)
*7: Gafquat 734, by Ashland Specialty Ingredients, modified alcohol solution of Polyquaternium-11 (50 mass%), polymer Tg of 70°C
*8: Marukasol R, by Maruzen Petrochemical Co., Ltd., isododecane
*9: Black BL-100P, by Miyoshi Kasei, Inc., iron oxide

As in Table 1, it can be seen that when the cosmetic composition of the present invention is applied to hair, it can impart a smooth feel, and the composition is also excellent in ease of applying and spreading as well as washing durability.

Industrial Applicability

According to the present invention, it is possible to provide a cosmetic composition that can impart a smooth feel when applied to keratin substances such as skin and hair, has excellent washing durability, and is easy to spread over a wide area. Further, when the cosmetic composition is used as a hair dye composition, it can impart a smooth feel and ease of applying and spreading to the hair with good color duration and little discoloration by shampooing.

## Claims

**1.** A cosmetic composition comprising

a component (A): a film-forming polymer comprising an M unit represented by $(R^1)_3SiO_{1/2}$ and a Q unit represented by $SiO_{4/2}$ (wherein $R^1$ represents a hydrocarbon group having 1 or more and 12 or less carbon atoms and optionally substituted with fluorine, or a hydroxy group, and plural $R^1$'s can be the same as or different from each other),
a component (B): a film-forming polymer other than the component (A), and
a component (C): a volatile solvent,

wherein the component (C) has a content of 50% by mass or more.

**2.** The cosmetic composition according to claim 1, wherein the component (B) is one or more selected from the group consisting of the following components (B 1) to (B6):

(B1) a silicone resin comprising a T unit represented by $R^1SiO_{3/2}$ and substantially not containing a Q unit represented by $SiO_{4/2}$ ($R^1$ is the same as above)
(B2) an acrylic silicone polymer
(B3) a silicone-modified alicyclic structure-containing polymer
(B4) a silicone-modified pullulan
(B5) a polyurea/urethane silicone
(B6) a non-silicone-based polymer with a glass transition temperature of 200°C or lower.

**3.** The cosmetic composition according to claim 1 or 2, wherein the component (B) is one or more selected from the group consisting of the following component (B 1), component (B2), and component (B6):

(B1) a silicone resin comprising a T unit represented by $R^1SiO_{3/2}$ and substantially not containing a Q unit represented by $SiO_{4/2}$ ($R^1$ is the same as above)
(B2) an acrylic silicone polymer

(B6) a non-silicone-based polymer with a glass transition temperature of 200°C or lower.

**4.** The cosmetic composition according to any one of claims 1 to 3, wherein a ratio of a content by mass of the component (A) to a total content by mass of the component (A) and the component (B) in the cosmetic composition [(A)/{(A) + (B)}] is 10% or more and 90% or less.

**5.** The cosmetic composition according to any one of claims 1 to 4, wherein a total content of the component (A) and the component (B) in the cosmetic composition is 1% by mass or more and 40% by mass or less.

**6.** The cosmetic composition according to any one of claims 1 to 5, wherein the component (C) is one or more selected from the group consisting of an alcohol-based solvent, a hydrocarbon-based solvent, and a silicone-based solvent.

**7.** A hair cosmetic composition composed of the cosmetic composition according to any one of claims 1 to 6.

**8.** A hair dye composition composed of the cosmetic composition according to any one of claims 1 to 6.

**9.** A method for treating a keratin substance, comprising a step of applying the cosmetic composition according to any one of claims 1 to 6 to a keratin substance and then drying it.

**10.** A method for treating hair, comprising a step of applying the hair cosmetic composition according to claim 7 to hair and then drying it.

**11.** A method for dyeing hair, comprising a step of applying the hair dye composition according to claim 8 to hair and then drying it.

**12.** A cosmetic kit provided with two or more compositions,
wherein the following components (A) to (C) are contained in a cosmetic composition obtained by mixing the compositions:

a component (A): a film-forming polymer comprising an M unit represented by $(R^1)_3SiO_{1/2}$ and a Q unit represented by $SiO_{4/2}$ (wherein $R^1$ represents a hydrocarbon group having 1 or more and 12 or less carbon atoms and optionally substituted with fluorine, or a hydroxy group, and plural $R^1$'s can be the same as or different from each other),
a component (B): a film-forming polymer other than the component (A), and
a component (C): a volatile solvent.

**INTERNATIONAL SEARCH REPORT**

International application No.
**PCT/JP2022/028785**

**A. CLASSIFICATION OF SUBJECT MATTER**

***A61Q 5/06***(2006.01)i; ***A61Q 5/12***(2006.01)i; ***A61K 8/31***(2006.01)i; ***A61K 8/34***(2006.01)i; ***A61K 8/81***(2006.01)i; ***A61K 8/891***(2006.01)i
FI: A61K8/891; A61K8/81; A61K8/34; A61K8/31; A61Q5/06; A61Q5/12

According to International Patent Classification (IPC) or to both national classification and IPC

**B. FIELDS SEARCHED**

Minimum documentation searched (classification system followed by classification symbols)

A61Q5/06; A61Q5/12; A61K8/31; A61K8/34; A61K8/81; A61K8/891

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

Mintel GNPD

**C. DOCUMENTS CONSIDERED TO BE RELEVANT**

| Category* | Citation of document, with indication, where appropriate, of the relevant passages | Relevant to claim No. |
|---|---|---|
| X | SCHWARZKOPF & HENKEL. Germany. Blow & Go Express Blow-Dry Spray. Mintel GNPD [online]. February 2017, Internet <URL: https://www.portal.mintel.com>, ID#4640175, [retrieval date 07 September 2022], product details, product information, item description<br>product details, product information, item description | 1-7, 9-10 |
| A | | 8, 11-12 |
| X | JP 2020-075881 A (SHIN-ETSU CHEMICAL CO., LTD.) 21 May 2020 (2020-05-21)<br>claims, paragraph [0103], example 28 | 1-6 |
| A | | 7-12 |
| A | JP 2021-506736 A (LVMH RECHERCHE) 22 February 2021 (2021-02-22)<br>entire text | 1-12 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **12 September 2022** | **27 September 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br><br>Telephone No. |

Form PCT/ISA/210 (second sheet) (January 2015)

**INTERNATIONAL SEARCH REPORT**
**Information on patent family members**

International application No.
**PCT/JP2022/028785**

| Patent document cited in search report | Publication date (day/month/year) | Patent family member(s) | Publication date (day/month/year) |
|---|---|---|---|
| JP 2020-075881 A | 21 May 2020 | US 2022/0000756 A1<br>claims, paragraphs [0204]-[0206], example 28<br>WO 2020/095757 A1<br>EP 3878517 A1<br>CN 112955223 A | |
| JP 2021-506736 A | 22 February 2021 | US 2020/0138692 A1<br>entire text<br>WO 2019/115949 A1<br>EP 3723708 A1<br>CN 110730653 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2015515981 A
- JP H10265354 A
- JP H111530 A
- JP 2000063225 A
- JP H225411 A
- JP H2132141 A
- JP H3162442 A
- JP 2003104825 A
- JP H692825 A